Europäisches Patentamt

European Patent Office

Office européen des brevets

(19)

(11) **EP 0 994 351 A1**

(12) **DEMANDE DE BREVET EUROPEEN**

(43) Date de publication:
**19.04.2000 Bulletin 2000/16**

(51) Int Cl.⁷: **G01N 33/14**

(21) Numéro de dépôt: **99402485.9**

(22) Date de dépôt: **11.10.1999**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **13.10.1998 FR 9812788**

(71) Demandeur: **Flotech 2000**
**38000 Grenoble (FR)**

(72) Inventeurs:
- **Meurisse, Jacques**
  **38410 Saint Martin d'Uriage (FR)**
- **Casabianca, Hervé**
  **69100 Villeurbanne (FR)**

(74) Mandataire: **Robert, Jean-Pierre et al**
**Cabinet Patco.**
**22, rue du Général Foy**
**75008 Paris (FR)**

(54) **Procédé de détection d'une adultération d'un jus de fruit concentré par des sirops de sucres exogènes**

(57) L'invention concerne un procédé de détection de l'adultération d'un jus de fruit concentré par ajout de sirops de sucres exogènes, selon lequel on procède à une chromatographie capillaire gazeuse du jus de fruit selon une méthode classique précédée d'une préparation et associée à une détection connues en elles-mêmes, procédé qui consiste à retenir dans le chromatogramme du produit les pics relatifs aux oligosaccharides marqueurs des sirops de sucres exogènes après les avoir quantifiés relativement à un ensemble prédéterminé d'oligosaccharides, à les introduire dans une banque de données comportant notamment les valeurs quantifiées de chromatographies antérieures de purs jus de fruits concentrés et ayant subi le traitement de conservation préalable à leur mise sur le marché et de sirops de sucres également traités, et à procéder à un traitement par analyse statistique de la banque de données ainsi enrichie pour situer le jus de fruit en cause parmi les produits antérieurement analysés.

FIG.5

## Description

La présente invention concerne un procédé de détection de la fraude dans l'élaboration de jus de fruit concentrés.

Les jus de fruits concentrés, notamment les jus de fruits rouges, entrent dans la composition de nombreux produits alimentaires, en particulier desserts, crèmes glacées, entremets aux fruits.

On sait aujourd'hui, de manière certaine, détecter l'ajout d'un colorant ou d'un agent gustatif étranger au fruit. Il est beaucoup plus délicat de détecter l'ajout de sirop de sucres exogènes.

Il existe actuellement sur le marché de nombreux produits appelés "pur jus de fruits concentrés" qui sont en réalité de savants mélanges de concentrés de fruits et de sirops de sucres exogènes. Cette pratique trouve sa justification dans le fait que les produits ainsi obtenus sont de coût moins élevé que les purs jus concentrés. En effet, les sirops de sucres proviennent du traitement du maïs, de la betterave ou de fruits dont la production est abondante et bon marché.

Il résulte de cette pratique une concurrence entre les produits authentiques et les produits adultérés, concurrence qui est déloyale puisque les utilisateurs de ces produits ne sont pas à même de distinguer de manière simple entre les deux types de produits, et donc privilégient le plus souvent les produits meilleur marché. En effet, les méthodes d'analyse portant sur les sucres, bien que de plus en plus raffinées, comportent pour chacune des incertitudes qui ne permettent pas de détecter à coup sûr la fraude. On augmente le degré de certitude en utilisant plusieurs méthodes d'analyse mais il s'agit alors d'une opération qui devient coûteuse pour l'utilisateur de jus concentrés.

L'une des méthodes aujourd'hui pratiquées est l'analyse des sucres par chromatographie capillaire gazeuse. Cette méthode mise au point par l'équipe du professeur canadien Nicholas LOW est assez élégante et fait apparaître dans les chromatogrammes des pics correspondant à certains oligosaccharides dont on sait qu'un pur jus de fruit est dépourvu. Ainsi la présence d'un ou plusieurs pics de ce type dans un chromatogramme de concentré de jus de fruit laisse à penser que le produit analysé a été coupé par un sirop de sucres.

Ce n'est cependant qu'une présomption car on s'est aperçu que certains concentrés purs pouvaient aussi présenter de tels pics, notamment après avoir subi les traitements biologiques appropriés qui sont nécessaires à la conservation longue durée des produits (enzymation et pasteurisation). Ces traitements modifient le profil chromatographique des sucres présents dans les jus concentrés, notamment en ce qui concerne les oligosaccharides.

Le but de l'invention est de perfectionner cette méthode par chromatographie gazeuse afin de supprimer autant que faire se peut le recours à l'interprétation visuelle des chromatogrammes.

A cet effet, l'invention a donc pour objet un procédé de détection de l'adultération d'un jus de fruit concentré par ajout de sirops de sucres exogènes, selon lequel on procède à une chromatographie capillaire gazeuse du jus de fruit selon une méthode classique précédée d'une préparation et associée à une détection connues en elles-mêmes. Selon l'invention, le procédé consiste à retenir dans le chromatogramme du produit, les pics relatifs aux oligosaccharides marqueurs des sirops de sucres exogènes après les avoir quantifiés relativement à un ensemble prédéterminé d'oligosaccharides, à les introduire dans une banque de données comportant notamment les valeurs quantifiées de chromatographies antérieures de purs jus de fruits concentrés et ayant subi les traitements de conservation préalable à leur mise sur le marché et de sirop de sucres également traités pour leur conservation et à procéder à un traitement par analyse statistique de la banque de données ainsi enrichie pour situer le produit en cause parmi les produits antérieurement analysés.

D'autres caractéristiques et avantages de l'invention ressortiront de la description donnée ci-après de quelques exemples de sa réalisation.

Il sera fait référence aux dessins annexés dans lesquels :

- la figure 1 est un chromatogramme d'un jus de framboise pur concentré,
- la figure 2 est la zone agrandie de ce chromatogramme au niveau des disaccharides,
- la figure 3 illustre la zone des disaccharides d'un chromatogramme de sirop de sucres,
- les figures 4 et 5 sont des graphes illustrant l'analyse en composante principale (ACP) respectivement de jus de fraise et de jus de framboise, dont certains sont naturels et certains adultérés.

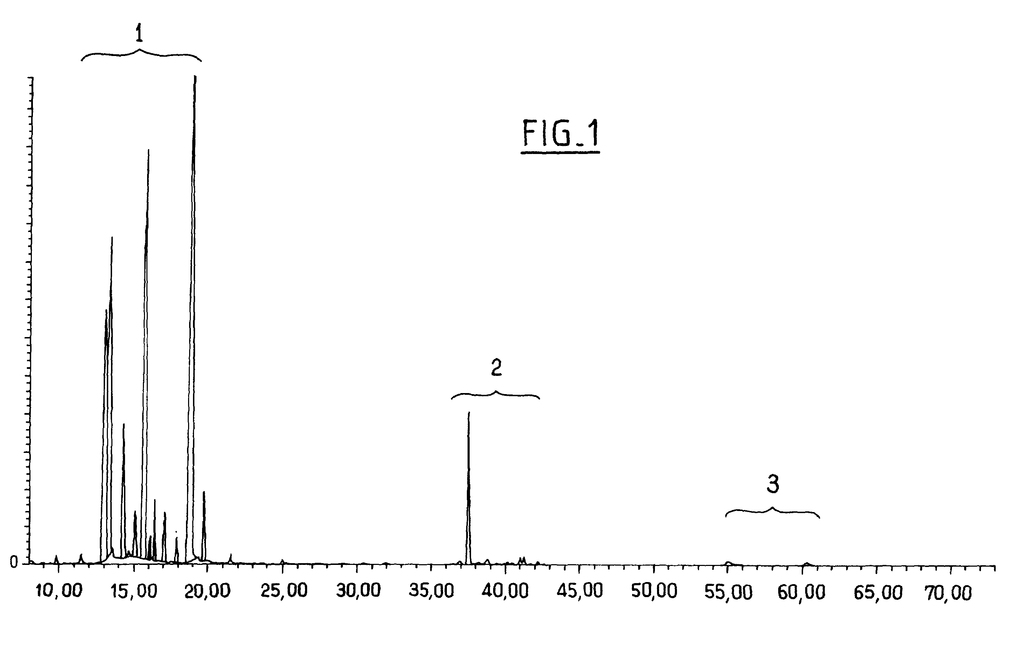

Le chromatogramme de la figure 1 est l'illustration de la composition et de la teneur en sucres selon la forme prise par ces sucres d'un jus de framboise concentré et traité par pasteurisation et enzymation en vue de sa conservation.

Ce chromatogramme comporte en abscisse une échelle de temps de rétention (temps d'élution) et en ordonnée une échelle indiquant l'abondance du sucre identifié par son temps d'élution. Le graphe est une succession de pics dont l'aire est proportionnelle à la quantité de chaque produit élué. On comprend sur ce graphe que les sucres en plus grande quantité sont les monosaccharides (glucose, fructose) qui donnent naissance aux pics de la zone 1 et dont les temps d'élution sont les plus brefs. On remarque ensuite une zone 2 où les pics, à l'exception d'un seul, sont à peine marqués à l'échelle du graphe. Il s'agit de la zone des disaccharides, le pic le plus important représentant le saccharose.

La zone 3 est la zone des trisaccharides qui, bien entendu, sont encore moins abondants que les sucres précédents et qui ont des temps d'élution encore plus élevés. Il existe bien évidemment d'autres polymères (polysaccharides) qui n'apparaissent pas sur ce chromatogramme.

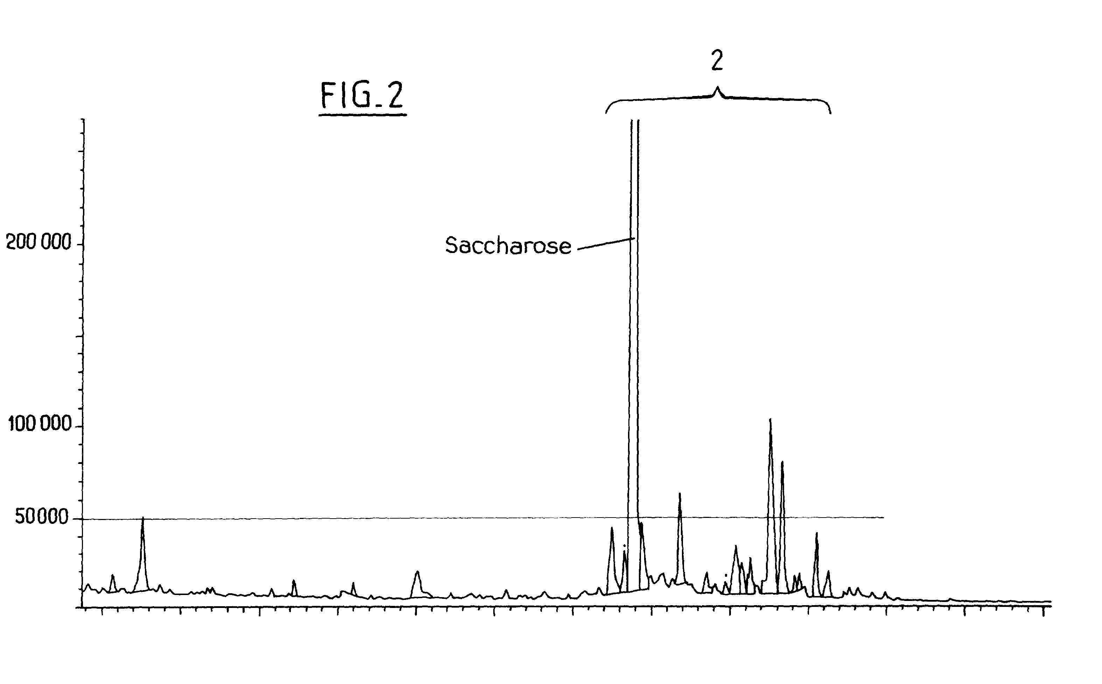

A la figure 2, la zone 2 des disaccharides du chromatogramme de la figure 1 a été agrandie. Cette partie agrandie fait apparaître un grand nombre de pics de diverses tailles qui tous correspondent à un disaccharide dont on peut apprécier l'abondance comparativement aux autres disaccharides et à l'exception du saccharose qui est éliminé, en mesurant l'aire du pic qui lui correspond et en la rapportant à l'aire totale de tous les disaccharides. Ainsi un disaccharide est-il caractérisé dans un produit par son temps (moment) d'élution et par son pourcentage. Le temps ou moment d'élution évolu principalement en fonction du vieillissement de la colonne d'élution de l'appareil.

Afin de s'affranchir de cette dérive, on utilise une procédure de calibration avant chaque campagne quotidienne de chromatographie, consistant à faire passer dans l'appareil un mélange de paraffines dont on connaît parfaitement la chaîne carbonée, ce passage étant effectué dans les mêmes conditions que les chromatographies de la campagne qui suivra (même température, même débit,...). A chaque pic correspond un moment d'élution auquel un indice est affecté (indice de Kovats) soit 1900 pour une chaîne à 19 carbones. Après la calibration, le moment d'élution par exemple d'un disaccharide, sera traduit en un indice de Kovats directement par l'appareil, indice calculé par la formule suivante :

$$I_x = (T_x - T_n)/(T_{n+1} - T_n) \times 100 + 100 \times n$$

où, $T_x$ est le temps de rétention du sucre en cause,
$T_n$ est le temps de rétention de la paraffine immédiatement inférieur au temps $T_x$,
$T_{n+1}$ est le temps de rétention de la paraffine immédiatement supérieur au temps $T_x$,
et n est le nombre de carbones de la paraffine éluée au temps $T_n$.

On comprend qu'ainsi pour chaque chromatographie (associée à une détection en aval du type spectrométrie de masse ou ionisation de flamme) les pics se traduisent par deux valeurs numériques, un indice de Kovats et un pourcentage.

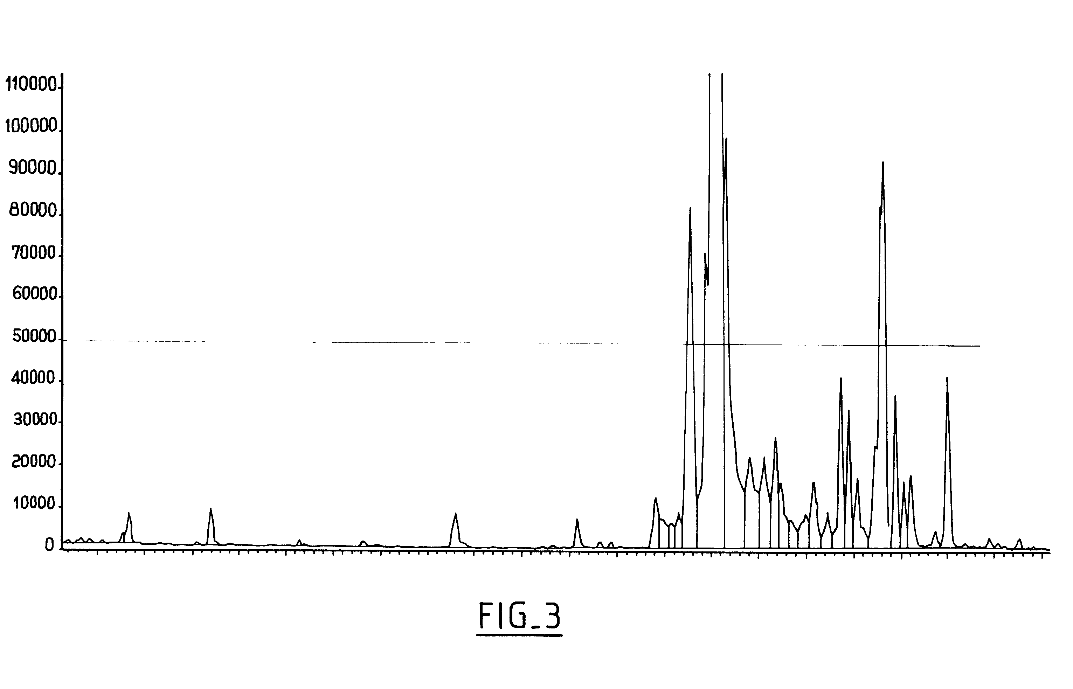

La figure 3 est le chromatogramme d'un sirop de sucre tel qu'il peut être employé pour couper des jus de fruits naturels, chromatogramme limité au niveau des disaccharides. Dans ce graphe, on constate également la présence majoritaire de saccharose et celle d'autres disaccharides dont les temps d'élution sont bien entendu situés dans la même zone que ceux des sucres de même ordre d'un pur jus de fruit. On comprend au vu des figures 2 et 3 qu'il devient très difficile de comparer visuellement ces deux zones, même établies à échelle identique et même avec une grande habitude, de sorte que de nombreuses incertitudes sont à lever quand il s'agit de décider de la présence ou de l'absence d'un sucre exogène dans un jus de fruit. Outre la complexité des chromatogrammes à comparer, il faut ajouter le fait que les temps d'élution ont pu varier d'un chromatogramme à l'autre pour un même produit, ce qui augmente encore les difficultés d'interprétation.

Pour lever ces incertitudes, le procédé de l'invention consiste tout d'abord à déterminer les marqueurs des différents sirops de sucre en usage dans le commerce, c'est-à-dire à choisir un ou quelques uns des oligosaccharides les plus abondants dans chacun des sirops (alors qu'ils sont absents ou peu abondants dans les purs jus de fruits concentrés). Ces marqueurs sont identifiés par leur indice (Kovats) et leur pourcentage pour être mémorisés dans une banque de données.

On procède ensuite à la chromatographie de purs jus de fruits dont on est certain qu'il n'ont pas été adultérés et on extrait de chaque chromatogramme les couples de valeurs (indices et pourcentages) correspondants aux marqueurs choisis. Ces valeurs sont également logées dans la banque de données. A titre d'exemple, le tableau ci-dessous illustre quelques relevés réels de l'abondance (en pourcentage) de chacun des huit marqueurs M1 à M8 (huit oligosaccharides) identifiés par leur indice dans, d'une part six sirops de sucre connus S1 à S6 et d'autre part, neuf jus de fruits purs concentrés pasteurisés et/ou enzymés F1 à F9 tels que ces produits sont sur le marché prêts à l'emploi.

|  | M1 | M2 | M3 | M4 | M5 | M6 | M7 | M8 |
|---|---|---|---|---|---|---|---|---|
| S1 | 0 | 6,155 | 7,095 | 1,964 | 6,49 | 11,406 | 1,967 | 2,136 |
| S2 | 0 | 7,261 | 0 | 2,064 | 5,109 | 13,891 | 2,029 | 3,472 |
| S3 | 0,091 | 0,62 | 1,283 | 7,181 | 1,699 | 2,02 | 11,577 | 14,292 |

(suite)

|  | M1 | M2 | M3 | M4 | M5 | M6 | M7 | M8 |
|---|---|---|---|---|---|---|---|---|
| S4 | 0,856 | 8,547 | 7,537 | 6,375 | 9,063 | 8,801 | 0,579 | 0,134 |
| S5 | 22,793 | 4,074 | 4,057 | 29,519 | 0,691 | 0,492 | 0,037 | 0 |
| S6 | 0,459 | 15,93 | 20,633 | 5,506 | 4,755 | 13,055 | 0 | 0 |
| F1 | 0 | 3,172 | 2,53 | 1,524 | 0 | 0,734 | 0 | 0 |
| F2 | 0,176 | 1,464 | 1,005 | 1,911 | 2,379 | 0 | 0,738 | 0,636 |
| F3 | 0 | 1,079 | 1,126 | 1,381 | 2,535 | 0 | 0,827 | 0,431 |
| F4 | 0 | 1,291 | 1,264 | 1,515 | 2,71 | 0 | 2,507 | 0,553 |
| F5 | 0,25 | 1,59 | 1,23 | 1,408 | 2,347 | 0 | 0,396 | 0,069 |
| F6 | 0 | 1,698 | 0,942 | 1,625 | 2,181 | 0 | 0,515 | 0,234 |
| F7 | 0 | 1,95 | 0,879 | 1,644 | 2,742 | 0 | 0,846 | 0,107 |
| F8 | 0 | 3,563 | 0 | 1,252 | 0 | 0 | 0,136 | 0,209 |
| F9 | 0 | 2,589 | 0 | 1,791 | 0 | 0 | 0,183 | 0,285 |

Dans le tableau ci-dessus, les fruits sont de même nature, par exemple des fraises, et les marqueurs M1 à M8 ont été choisis pour leur forte abondance dans les sirops de sucre et leur faible présence dans les fruits naturels. Bien entendu la banque de données pourra comprendre des registres affectés aux fruits de même nature (fraises, framboises, cassis, pommes, agrumes, fruits à noyaux,...) et aux marqueurs les mieux adaptés à la nature des fruits analysés. Ainsi, l'analyse statistique de la banque de données pourra porter sur le registre correspondant à une famille de jus de fruits à analyser au moyen de marqueurs adaptés.

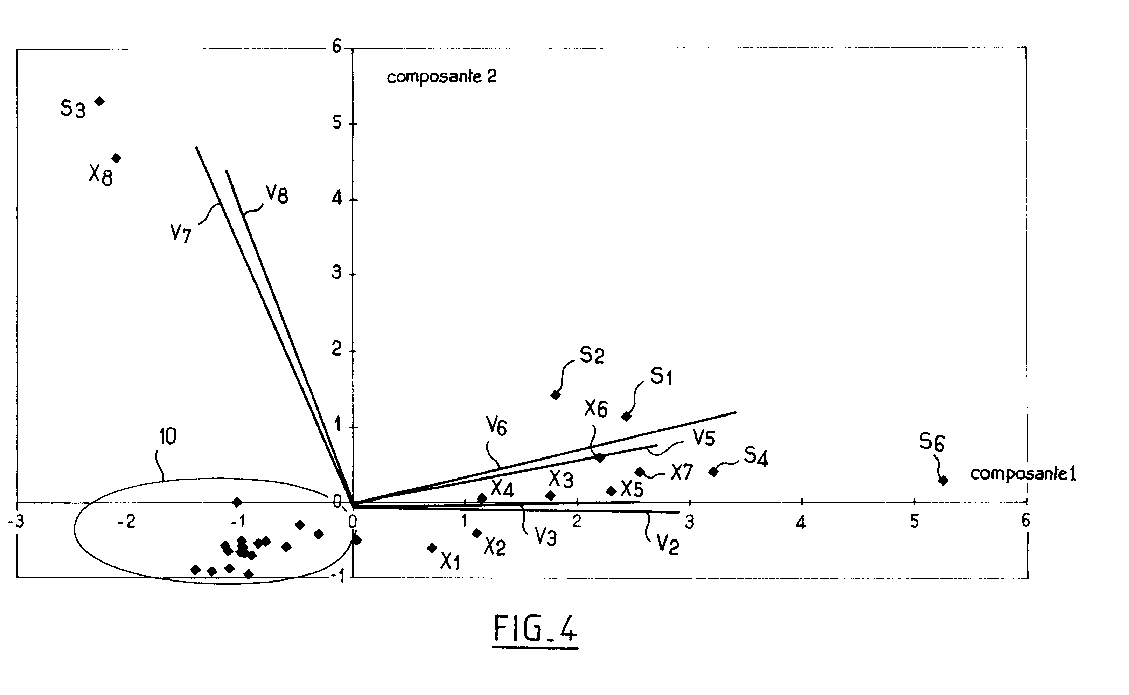

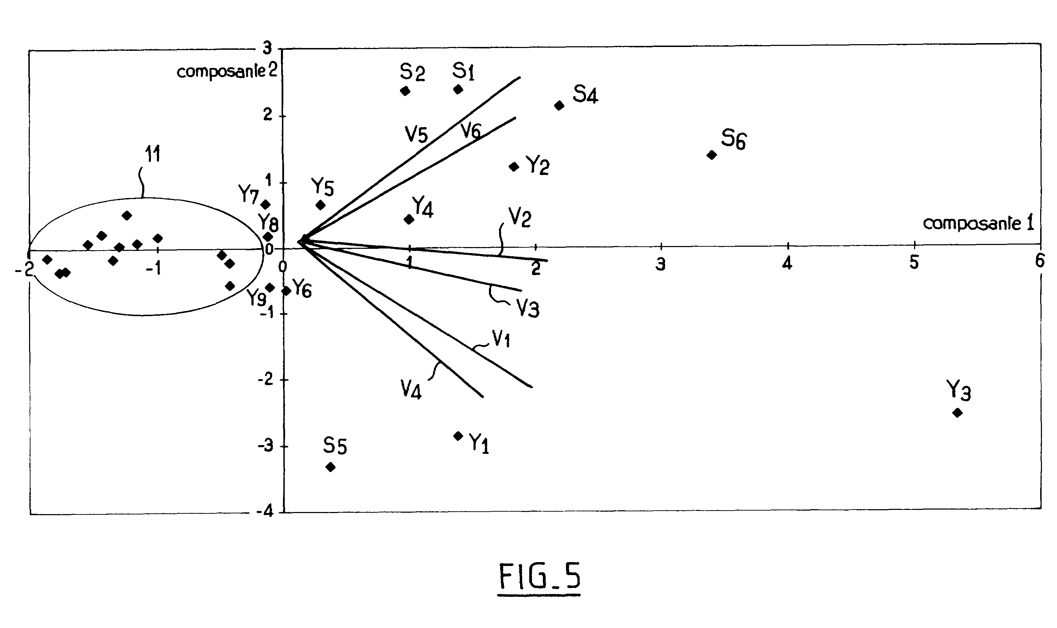

Cette analyse statistique est fondée sur l'une ou l'autre des méthodes d'analyse connues en elles-mêmes ayant pour but de permettre une représentation graphique plane simple de la banque de données (ou du registre concerné) à partir de laquelle il est possible de décider simplement si le produit analysé a été ou non adultéré. L'une de ces méthodes, l'analyse en composante principale (A C P) conduit aux graphes des figures 4 et 5.

La figure 4 est la représentation de l'analyse en composante principale d'un registre de la banque de données comprenant des échantillons de pur jus de fraise représentés par une zone 10 de points groupés, cinq des six sirops de sucres marqués S1, S2, S3, S4, S6 et quelques jus inconnus repérés X1 à X8. Les paramètres d'analyse sont constitués par les six marqueurs M2, M3, M5, M6, M7, M8. Les marqueurs M1 et M4 ont été retirés car ils correspondent au sirop de sucre S5 qui lui aussi a été retiré. Cette soustraction résulte du fait que le sirop S5 a un poids dans la représentation opposé à celui du sirop S3. En conservant ces deux sirops, l'analyse statistique aurait été de mauvaise qualité et de faible efficacité. Cette efficacité est mesurée par tests, inclus dans la méthode elle-même, qui la calcule automatiquement et guide l'opérateur quant au choix des paramètres à conserver. Sur ce graphique enfin, les vecteurs V2, V3, V5, V6, V7 et V8 représentent le poids des marqueurs paramètres respectivement M2, M3, M5, M6, M7 et M8 par rapport à tous les échantillons analysés. On remarque que la zone 10 est totalement opposée à ces vecteurs, ce qui signifie que ces paramètres ont peu d'influence sur des produits non fraudés.

En revanche, on constate que les produits X sont à l'extérieur de la zone 10 et dans la zone du graphe où les paramètres M2, M3, M5, M6 ont un poids important, ce qui correspond aux divers sirops S2, S1, S4, S6 ou, pour le produit X8, dans une zone où les paramètres M7 et M8 sont de grande importance, ce qui correspond à la présence d'un sirop S3.

La figure 5 est une représentation semblable à celle de la figure 4 pour un registre de la banque de données comprenant les échantillons de purs jus de framboise représentés par une zone 11 de points groupés, cinq des six sirops de sucres marqués S1, S2, S4, S5, S6 et quelques jus inconnus repérés Y1, Y9. Comme dans le cas précédent, afin d'obtenir une efficacité correcte, on a soustrait à l'ACP le sirop S3 qui est opposé au sirop S5 ainsi que les marqueurs M7 et M8 les plus abondants dans ce sirop. De la même manière que pour le graphe précédent, on constate que la zone 11 est opposée au vecteur des marqueurs M1 et M6 et qu'au moins les produits Y1, Y2, Y4 ont fait manifestement l'objet de coupage avec pour Yl des sirops de type S5 et pour Y2 des sirops de type S6.

D'autres analyses incorporant des produits volontairement adultérés avec des quantités relatives de sirops de sucres connues ont montré que la représentation des figures 4 et 5 peut être graduée et que la distance des points au centre du graphe est représentative le long des vecteurs, de l'importance de coupage par le sirop correspondant à ces vecteurs.

**Revendications**

1. Procédé de détection de l'adultération d'un jus de fruit concentré par ajout de sirops de sucres exogènes, selon lequel on procède à une chromatographie capillaire gazeuse du jus de fruit selon une méthode classique précédée d'une préparation et associée à une détection connues en elles-mêmes, caractérisé en ce qu'il consiste à retenir, dans le chromatogramme du jus de fruit, les pics relatifs aux oligosaccharides marqueurs des sirops de sucres exogènes après les avoir quantifiés relativement à un ensemble prédéterminé d'oligosaccharides, à les introduire dans une banque de données comportant notamment les valeurs quantifiées de chromatographies antérieures de purs jus de fruits concentrés et ayant subi le traitement de conservation préalable à leur mise sur le marché et de sirops de sucres également traités, et à procéder à un traitement par analyse statistique de la banque de données ainsi enrichie pour situer le jus de fruit en cause parmi les produits antérieurement analysés.

2. Procédé de détection selon la revendication 1, caractérisé en ce qu'il comporte, avant chaque campagne de chromatographie, une étape de calibration de l'appareil au moyen de paraffines dont les chaînes carbonées sont connues et croissantes, permettant d'affranchir l'appareil de sa dérive dans le temps et de calculer un indice pour chaque pic, cet indice constituant le caractère identifiant de l'oligosaccharide auquel il correspond.

3. Procédé selon la revendication 1 ou la revendication 2, caractérisé en ce que l'analyse statistique des données de la banque de données est une analyse en composante principale (ACP).

4. Procédé selon l'une des revendications précédentes, caractérisé en ce que la détection associée à la chromatographie capillaire gazeuse est une détection par spectrographie de masse.

5. Procédé selon l'une des revendications précédentes, caractérisé en ce que la totalité des valeurs de la banque de données est acquise sur les produits ayant subi un traitement industriel connu (pasteurisation et/ou enzymation) pour sa conservation.

FIG.1

EP 0 994 351 A1

FIG.2

EP 0 994 351 A1

FIG_3

EP 0 994 351 A1

FIG. 4

FIG.5

EP 0 994 351 A1

**EP 0 994 351 A1**

Office européen
des brevets

**RAPPORT DE RECHERCHE EUROPEENNE**

Numéro de la demande

EP 99 40 2485

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | US 5 200 909 A (JUERGENS) 6 avril 1993 (1993-04-06) * abrégé * * colonne 11, ligne 22 - ligne 29; figure 10 * | 1-5 | G01N33/14 |
| A | PATENT ABSTRACTS OF JAPAN vol. 98, no. 5, 30 avril 1998 (1998-04-30) & JP 10 019849 A (SHIMADZU CORPORATION) * abrégé * | 1-5 | |
| A | US 4 102 646 A (SLEETER) 25 juillet 1978 (1978-07-25) * abrégé * * colonne 5, ligne 1 - colonne 6, ligne 6; figure 1 * | 1-5 | |

DOMAINES TECHNIQUES RECHERCHES (Int.Cl.7)

G01N

Le présent rapport a été établi pour toutes les revendications

| Lieu de la recherche | Date d'achèvement de la recherche | Examinateur |
|---|---|---|
| LA HAYE | 1 février 2000 | Kempf, G |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

11

EP 0 994 351 A1

**ANNEXE AU RAPPORT DE RECHERCHE EUROPEENNE
RELATIF A LA DEMANDE DE BREVET EUROPEEN NO.**                EP 99 40 2485

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

01-02-2000

| Document brevet cité au rapport de recherche | Date de publication | Membre(s) de la famille de brevet(s) | Date de publication |
|---|---|---|---|
| US 5200909 A | 06-04-1993 | AUCUN | |
| JP 10019849 A | 23-01-1998 | AUCUN | |
| US 4102646 A | 25-07-1978 | AUCUN | |